# EUROPEAN PATENT APPLICATION

(11) **EP 3 392 263 A1**
(43) Date of publication of application: **24.10.2018**
(21) Application number: 16874843.2
(22) Date of filing: 14.12.2016
(51) Int. Cl.: C07K 7/00, C07K 7/08, C07K 14/00, A61K 38/16, A61P 37/04

(54) **POLYPEPTIDE COMPOUND, PREPARATION METHOD THEREFOR AND USE THEREOF**

(30) Priority: 14.12.2015 CN 201510924004
(71) Applicant: Han, Su, Beijing 100080 (CN)
(72) Inventor: Han, Su, Beijing 100080 (CN)
(74) Representative: Hoffmann Eitle
(86) International application number: PCT/CN2016/109916
(87) International publication number: WO 2017/101786

(57) **Abstract**

The present invention discloses a polypeptide compound, a method for preparing the polypeptide compound, and an application of the polypeptide compound. The polypeptide compound is represented by a structural formula A-(A-K)ₙ-Y, n being a natural number; wherein A is a short peptide fragment with biological activity; K is lysine Fmoc-Lys (Dde)-OH that contains two active amino groups, and Y is null or any one or more amino acids or chemical groups; when n=1, the structural formula of the polypeptide compound is A-(A-K)-Y; when n=2, the structural formula of the polypeptide compound is A-(A-K)₂-Y; when n=3, the structural formula of the polypeptide compound is A-(A-K)₃-Y; .... The polypeptide compound disclosed and provided in the present invention has effects of inhibiting tumor growth and improving immune function.

## Description

### Field of the Invention

The present invention relates to the bio-pharmaceutical field, in particular to a polypeptide compound molecule, a preparation method of the polypeptide compound, and an application of the polypeptide compound in preparation of medicines for inhibiting tumor growth, improving immune function, and inhibiting vascularization.

### Background of the Invention

The genetic genes of organisms are stored in polydeoxynucleotide chains, and proteins that execute biological functions are coded in the genetic genes. Various proteins exist in organisms and they execute different biological functions to maintain vital activities. Though there are numerous kinds of proteins, they are essentially composed of 20 kinds of natural amino acids that exist in the natural world. Proteins differ significantly owing to the composition and sequence of amino acids. Generally speaking, molecules that contain 50 or more amino acids are referred to as proteins, peptide chains that contain 10 or more amino acids are referred to as polypeptides, and peptide chains that contain less than 10 amino acids are referred to as oligopeptides. The smallest functional small-peptide discovered up to now only contains 2 amino acids. Usually, functional small-peptides that are composed of 4 or more amino acids are commonly seen.

As the Human Genome Project has been finished and the Human Proteome Project has been developed, more and more functional protein segments will be discovered and applied as medicines in the bio-pharmaceutical field. A functional protein segment usually refers to a straight-fragment chain polypeptide fragment that is found as having a specific biological function. Such a functional protein segment usually is a peptide segment composed of two to dozens of amino acids. The identified and discovered functional protein segments can be prepared in an artificial synthesis approach. Polypeptide medicines that have been developed and applied clinically include "oxytocin", "thymosin α1", and "thymopentin", etc. Polypeptide medicines available presently include "octreotide", which is prepared through artificial modification of natural peptide chains and used to treat hemorrhage of digestive tract and acromegaly, and "hirudin peptide", which has an anti-coagulation effect. The functional segments in proteins often can be screened to polypeptide segments that contain dozens of amino acids or even as few as two amino acids. They set a basis for artificial synthesis and application of functional polypeptide segments.

In proteins, polypeptides or oligopeptides, the deletion, addition or substitution of a single amino acid, closing of amino terminal (N terminal) or carboxyl terminal (C terminal), addition of any chemical group into the sequence or at the free end, etc., will result in changes of the original biological activity of the proteins, polypeptides, or oligopeptides. Designing, screening, and discovering new functional peptide fragments or seeking for efficient peptide fragments is an important link in the development of medicines.

### Contents of the Invention

To overcome the technical defects in the prior art, in one aspect, the present invention provides a multi-copy polypeptide compound represented by structural formula A-(A-K)ₙ-Y, n being a natural number;
Wherein, A is a short peptide fragment with biological activity; K is lysine Fmoc-Lys (Dde)-OH that contains two active amino groups, n is the number of K, Y is null or any one or more amino acids or chemical groups; when n=1, the structural formula of the polypeptide compound A is A-(A-K)-Y; when n=2, the structural formula of the polypeptide compound A is A-(A-K)₂-Y; and when n=3, the structural formula of the polypeptide compound A is A-(A-K)₃-Y; ...;
A preferably is a short peptide fragment X_{A}X_{B}X_{C}X_{D}-X; when A is X_{A}X_{B}X_{C}X_{D}-X, the structural formula of the polypeptide compound is X_{A}X_{B}X_{c}X_{D}-X-(X_{A}X_{B}X_{C}X_{D}-X-K)ₙ-Y; wherein, X_{A} and X_{B} are one or two of aliphatic amino acids, X_{C} and X_{D} are selected from one or two of aliphatic amino acids and aromatic heterocyclic amino acids, and X is null or any one or more amino acids or chemical groups.

When n=2 and A is X_{A}X_{B}X_{C}X_{D}-X, the structural formula of the polypeptide compound is X_{A}X_{B}X_{C}X_{D}-X-(X_{A}X_{B}X_{C}X_{D}-X-K)₂-Y, as shown in formula 3:

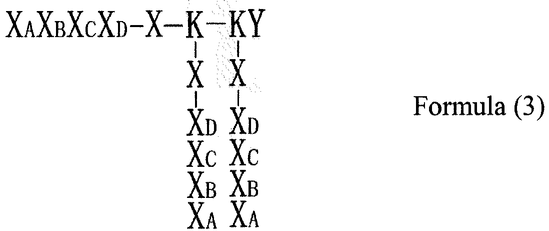

When n=4 and A is X_{A}X_{B}X_{C}X_{D}-X, the structural formula of the polypeptide compound is X_{A}X_{B}X_{C}X_{D}-X-(X_{A}X_{B}X_{C}X_{D}-X-K)₄-Y, as shown in formula 4:

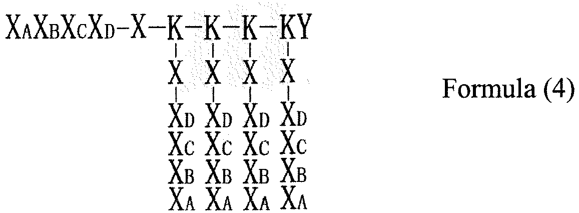

Where, X or Y is null, or any amino acid, or a peptide fragment composed of any number of amino acids, or a chemical group that can connect amino acids or peptide fragments, and X and Y may be the same or different from each other; for example, X may be null, and Y may be glycine (Gly, G).

X_{A} and X_{B} are aliphatic amino acid molecules, including any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), and glutamine (Gln, Q), and may be the same or different.

X_{C} and X_{D} are selected from any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), glutamine (Gln, Q), tryptophan (Trp, W), histidine (His, H) and proline (Pro, P), and may be the same or different.

The polypeptide compound further includes a salt compound formed by the polypeptide compound with an organic acid or inorganic acid.

The polypeptide compound further includes an ether, ester, glucoside, or glycoside compound, etc., which may be formed by the hydroxyl included in the polypeptide compound, but is not limited to compounds formed in such a way.

The polypeptide compound further includes a thioether or thioglycoside compound, which may be formed by the sulfhydryl, included in the polypeptide compound, or a compound containing disulfide bonds, which may be formed by the sulfhydryl included in the polypeptide compound with cysteine or peptide containing cysteine, but is not limited to compounds formed in such a way.

The polypeptide compound further includes an acylate or alkylate compound, which may be formed by the amido included in the polypeptide compound, or a glucoside compound, etc., which may be formed by the amido included in the polypeptide compound with saccharides, but is not limited to compounds formed in such a way.

The polypeptide compound further includes an ester or amide compound, etc., which may be formed by the carboxyl, included in the polypeptide compound, but is not limited to compounds formed in such a way.

The polypeptide compound further includes a glucoside, acylate, or alkylate compound, etc., which may be formed by the imino group included in the polypeptide compound, but is not limited to compounds formed in such a way.

The polypeptide compound further includes an ester, ether, glucoside, or glycoside compound, which may be formed by the phenolic hydroxyl included in the polypeptide compound, or a salt compound, which may be formed by the phenolic hydroxyl included in the polypeptide compound with organic alkalis or inorganic alkalis, but is not limited to compounds formed in such a way.

The polypeptide compound further includes a coordinate, clathrate, or chelate compound formed by the polypeptide compound with metal ions.

The polypeptide compound further includes a hydrate or solvent formed by the polypeptide compound.

In a second aspect, the present invention provides a pharmaceutical composition that contains the above-mentioned polypeptide compound, a geometrical isomer of the pharmaceutical composition, a pharmaceutically acceptable salt or solvated compound of the pharmaceutical composition, and the pharmaceutical composition in a form of pharmaceutical carrier or excipient.

In a third aspect, the present invention provides a method for preparing the above-mentioned polypeptide compound, wherein the synthetic route of A-(A-K)ₙ-Y is shown in formula 5,

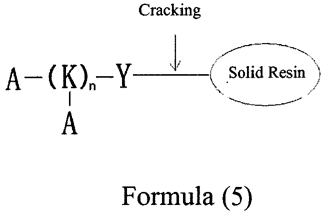

A preferably is a short peptide fragment X_{A}X_{B}X_{C}X_{D}-X; when A is X_{A}X_{B}X_{C}X_{D}-X, the structural formula of the polypeptide compound is X_{A}X_{B}X_{c}X_{D}-X-(X_{A}X_{B}X_{C}X_{D}-X-K)ₙ-Y; wherein, X_{A} and X_{B} are one or two of aliphatic amino acids, X_{C} and X_{D} are selected from one or two of aliphatic amino acids and aromatic heterocyclic amino acids, and X is null or any one or more amino acids or chemical groups;

First, Y is fixed to a solid resin to obtain Y-solid resin, the Y-solid resin is treated by Fmoc deprotection and the n pieces of Fmoc-Lys (Dde)-OH are condensed one by one, to accomplish preparation of a Kₙ-Y-solid resin peptide skeleton; then, the Kₙ-Y-solid resin peptide skeleton is treated by deprotection of the side chain amino group Dde and Fmoc, and segment A extension and introduction is carried out synchronously on the free amino groups of Kₙ-Y-solid resin, to obtain A-(A-K)ₙY-solid resin; after the synthesis is completed, A-(A-K)ₙY is cracked from the solid resin to obtain a crude peptide product, and the crude peptide product is purified by high efficiency liquid chromatography to obtain the polypeptide compound A-(A-K)ₙY.

An appropriate resin for preparation is selected according to the characteristics of the carboxyl terminal of the peptide product; "WANG resin" is selected if the carboxyl terminal of the peptide product is a free carboxyl group; "Rink resin" is selected if the carboxyl terminal of the synthetic peptide is an amido group; "CTC resin" is selected if the carboxyl terminal of the peptide product is Cys, Pro, or His.

The polypeptide compound is prepared with a solid-phase polypeptide synthesis method, which comprises the following steps:
Step 1: preparing Y-solid resin: if Y is a single amino acid, Y-solid resin may be purchased directly;
   If Y is a short peptide that contains a plurality of amino acids, a solid phase Fmoc/tBu method is used, i.e., Fmoc-aa₁-Wang resin is used as a starting raw material, synthesis is carried out from the carboxyl terminal (C) to the amino terminal (N), the protecting group of Fmoc at the amino terminal is removed so that the amino terminal becomes a free amino group, and then the amino terminal is condensed with resin, till Y-solid resin is obtained;
   Namely, if Y is a single amino acid aa, "aa₁-solid resin" may be purchased directly. If Y is a short peptide that contains a plurality of amino acids, a solid phase Fmoc/tBu method is used, i.e., Fmoc-aa₁-Wang resin (the substitution value is 0.40mmol/g) is used as a starting raw material, synthesis is carried out from carboxyl terminal (C) to amino terminal (N), the protecting group of Fmoc at the amino terminal is removed with 20% Piperidine/DMF (V/V) so that the amino terminal becomes a free amino group (2 times, 10min. per time), and Fmoc-aa-OH/HOBt/DIC in quantity equivalent to three times of equivalent is used to condense with the resin, and the reaction time is 1h. After each step of reaction, the resin is washed with DMF for 6 times, the extent of reaction is detected by Kaiser Test, and the condensation is repeated once more if it is found that the condensation reaction is incomplete. Fmoc-aa₂-OH, Fmoc-aa₃-OH, ... are introduced sequentially, and the condensation reaction is repeated, till "Y-solid resin" is formed completely.
Step 2: preparing Kₙ-Y-solid resin peptide skeleton: the Y-solid resin is treated by Fmoc deprotection to expose the free amino group, and condensation is carried out with Fmoc-Lys (Dde)-OH (the condensation method is the same as that in the step 1); the condensation is repeated till n pieces of Fmoc-Lys (Dde)-OH are condensed (denoted as "cycle 1"), and thereby a Kₙ-Y-solid resin peptide skeleton is prepared;
   Namely, Fmoc deprotection is carried out to expose the free amino group, and condensation is carried out with Fmoc-Lys (Dde)-OH (the condensation method is described in the step 1); the condensation is repeated till n pieces of Fmoc-Lys (Dde)-OH are condensed (denoted as "cycle 1"), and thereby a Kₙ-Y-solid resin peptide skeleton is prepared.
Step 3: carrying out amino deprotection for the Kₙ-Y-solid resin: the side chain Dde of Lys and the protecting group of Fmoc in the Kₙ-Y-solid resin peptide skeleton are removed, to obtain a Kₙ-Y-solid resin in which the free amino groups on the side chains are liberated;
   Namely, the Dde of side chain Lys and the protecting group of Fmoc in the "Kₙ-Y-solid resin" peptide chain are removed with 2% Piperidine/DMF (V/V) (twice, 15min. each time), to obtain a Kₙ-Y-solid resin in which the free amino groups on the side chains are liberated.
Step 4: synthesizing segments A synchronously on the free amino groups of Kₙ-Y-solid resin: the amino acids are condensed one by one with the method describe in the step 1, till segments A are completed and A-(A-K)ₙY-solid resin is obtained (denoted as "cycle 2");
   Namely, Fmoc-aa₁-OH, Fmoc-aa₂-OH, ... are condensed one by one with the method describe in the step 1, till segments A are completed and A-(A-K)ₙY-solid resin is obtained (denoted as "cycle 2").
Step 5: after the synthesis is completed, A-(A-K)ₙY is cracked from the solid resin, and the protecting groups on the side chains are removed, to obtain a crude product A-(A-K)ₙY;
   Namely, after the synthesis is completed, A-(A-K)ₙY is cracked from the solid resin with 95% TFA/H₂O, and the protecting groups on the side chains are removed (cracked for 3h at 30□); the eluent that contains A-(A-K)ₙY is collected, a plenty of cold anhydrous ether is added so that A-(A-K)ₙY precipitates, and then the mixture is centrifuged. The mixture is washed with ether for several times and then is dried; thus a crude product A-(A-K)ₙY is obtained.
Step 6: purifying to obtain a fine product: the crude peptide is purified by high efficiency liquid chromatography (HPLC), to obtain the polypeptide compound A-(A-K)ₙY at purity>98%.
   Namely, the crude product A-(A-K)ₙY is purified with a high efficiency liquid chromatographic (HPLC) column (Model Daiso C18, 10µm, 100Å, 50×250mm), wherein, a mobile phase A is water solution that contains 0.05% trifluoroacetic acid and 2% acetonitrile, a mobile phase B is 90% acetonitrile/water, the flow rate is 25mL/min., the ultraviolet detection wavelength is 220nm, the eluting peak solution is collected and then dried by freeze drying; thus, a white flocculent polypeptide compound A-(A-K)ₙY at purity>98% is obtained.
Step 7: storage: the white flocculent polypeptide compound A-(A-K)ₙY obtained after freeze drying is stored in a dark and cold environment.

Whenever an amino acid is introduced and condensed, and each time after an amino acid is introduced and condensed between the "cycle 1" and the "cycle 2", a "Kaiser Test" is carried out to detect the content of free amino groups, and condensation is repeated once more if the condensation rate of the reaction is not high enough.

In a fourth aspect, the present invention provides an application of the above-mentioned polypeptide compound or a polypeptide compound prepared with the above-mentioned method in preparation of medicines for inhibiting tumor growth in human or animal bodies.

The tumor is a malignant solid tumor (or residual tumor after medical operation) or a non-solid tumor such as hematological tumor (including leukaemia and lymphoma) in a human body.

The tumor includes, but is not limited to sarcoma, liver cancer, colon cancer, lung cancer, stomach cancer, mammary cancer, and cervical cancer.

In a fifth aspect, the present invention provides an application of the above-mentioned polypeptide compound or a polypeptide compound prepared with the above-mentioned method in preparation of immune medicines or medicines for improving immune function for humans or animals.

The polypeptide compound disclosed in the present invention is hopeful to become an effective ingredient in a variety of medicines, and is applicable to medicines for preventing and treating many diseases. Especially, the polypeptide compound can be widely applied in preparation of medicines for improving immune capability and inhibiting tumor growth.

### Detailed Description of the Embodiments

The multi-copy functional segment peptide molecules disclosed in the past are prepared with Fmoc-Lys (Fmoc)-OH, and the number of copies of active peptide segments contained in the obtained multi-copy peptide molecules are incremented by 2ⁿ (n=1, 2, 3, ...); for example, functional polypeptide compound molecules that contain two-copy peptide molecules, or four-copy peptide molecules, or eight-copy peptide molecules, or sixteen-copy peptide molecules, etc., ..., are formed. In polypeptide compounds prepared in such a way, as the number of copies of active fragments is incremented in a geometric progression, the molecular weight is also incremented in a geometric progression. Since the difference in molecular weight between polypeptide compound molecules with different numbers of copies of peptide molecules in different progressions is very high, the molecular weight may be beyond an optimal range for actual application. In actual applications, an optimal peptide molecule form for actual application usually has to be determined through comprehensive evaluation with consideration of many factors, including the relation between the size of peptide molecule and the immunogenicity, relation between the peptide molecule and the reaction of target molecule, and input-output ratio of peptide molecule synthesis, etc.

In full consideration of the above-mentioned problems in peptide molecules, the present invention provides a polypeptide molecule that is more practical and may have any number of copies of peptide molecules, and a method for preparing the polypeptide molecule. The polypeptide molecule and the preparation method provided in the present invention overcome many drawbacks in the prior art, e.g., a multi-copy polypeptide molecule has to be prepared only by incrementing the number of copies of peptide molecules in a geometric progression at present.

In the present invention, a branched polypeptide molecule A-(A-K)ₙ-Y that contains any copy of segment A is designed and prepared, wherein, A is an active peptide segment with specific biological functions; K is lysine Fmoc-Lys (Dde)-OH that contains two active amino groups, and n is the number of K, Y is null or any one or more amino acids or chemical groups. The structural formula A-(A-K)ₙ-Y is described as follows: when the structural formula of the polypeptide compound is A-(A-K)-Y, i.e., n-1, the total number of copies of short peptide fragment A with biological activity in the polypeptide compound is n+1=2; when the structural formula is A-(A-K)₂-Y, i.e., n=2, the total number of copies of A in the polypeptide compound is 3; and so on.

In the present invention, A generally represents an active peptide segment that has specific biological functions. To describe the intention of the present invention more clearly, in the embodiments, A is embodied as X_{A}X_{B}X_{C}X_{D}-X. Wherein, X_{A} and X_{B} are aliphatic amino acid molecules, including any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), and glutamine (Gln, Q), and may be the same or different. X_{C} and X_{D} may be selected from any one of above-mentioned aliphatic amino acid molecules, and may be the same or different; or they may be selected from heterocyclic amino acids, including any of tryptophan (Trp, W), histidine (His, H) and proline (Pro, P), and may be the same or different; X is null, or any amino acid, or a peptide fragment composed of any number of amino acids, or a chemical group that can bond up amino acids and peptide fragments. By changing the species of X_{A}, X_{B}, X_{C} and X_{D}, the polypeptide compound provided in the present invention can be used to inhibit tumor growth and improve immune response capability of living bodies. Therefore a medicine that can be used in clinical applications (for humans or animals) for improving immune function and/or inhibit tumor growth can be developed.

In year 1963, an American scientist R. B. Merrifield invented a solid-phase synthesis method for extending a peptide chain by fixing the carboxyl terminal (C terminal) of amino acids in a target peptide to insoluble resin and controlling the amino terminal (N terminals) of amino acids bonded to the resin to have a condensation reaction with the carboxyl terminal of amino acids to be bonded; that is to say, the amino acids are condensed one by one starting from the carboxyl terminal (C terminal) of the polypeptide and extend continuously towards the amino terminal (N terminal) of the polypeptide segment. Therefore, when the condensation reaction of the amino acids is executed, the amido groups and side chain groups of the amino acids to be bonded must be protected to avoid reaction of them. At present, commonly used protection methods include t-butyloxycarboryl (Boc) protection method, fluorenylmethoxycarbonyl (Fmoc) protection method, and hydrazine hydrate (Dde) protection method. Therefore, whenever an amino acid has been bonded, a deprotection-condensation cycle must be executed (i.e., the amido group on the solid-phase carrier is deprotected first, and then has a condensation reaction with the carboxyl of the next target amino acid to be bonded among amino acids in excessive quantity, to extend the peptide chain). Through each cycle, i.e., amino acid condensation → washing → deprotection → neutralization → washing, a target amino acid is bonded, till a target peptide chain in required length is synthesized.

In the present invention, first, lysine (Lys, K) Fmoc-Lys (Dde)-OH that has two active amino groups is utilized to have a condensation reaction sequentially till a desired number of copies is reached (the number n of Fmoc-Lys (Dde)-OH molecules to be condensed is equal to the desired number of copies minus 1), and therefore a Kₙ-Y molecule skeleton composed of -Lys (Dde)- and fixed to a solid resin is obtain through synthesis. Then, the protecting groups, Dde and Fmoc, of amino groups on the side chains of lysine (Lys, K) on the "Kₙ-Y-solid resin" are removed to expose free amino groups, an a cycle of Fmoc-aa-OH amino-acid condensation reactions is initiated, till segments A are synthesized, and therefore "A-(A-K)ₙY-solid resin" is obtained. Next, the A-(A-K)ₙY is cracked from the solid resin with 95% TFA/H₂O and is purified, so that an A-(A-K)ₙY fine product is obtained. The number of copies (n+1) of segments with biological activity contained in the polypeptide molecule obtained finally is determined by the number of 'n'; namely, if n=2, a three-copy A-(A-K)₂-Y peptide molecule that contains three copies of segment A is obtained; if n=4, a five-copy A-(A-K)₄-Y peptide molecule that contains five copies of segment A is obtained; and so on, a multi-copy peptide molecules that contains four copies, six copies, or seven copies of segment A, ..., etc., can be obtained.

After the synthesis utilizing the WANG resin is finished, the peptide chain is cracked from the solid resin with a TFA method, to obtain an A-(A-K)ₙY molecule, which contains two copies, three copies, four copies, five copies, six copies, seven copies, or eight copies, ....

Though the present invention describes a multi-copy polypeptide compound molecule structure and a method for preparing the multi-copy polypeptide compound molecule structure, peptide compounds with the molecular skeleton A-(A-K)₄-Y synthesized with other methods are also included in the present invention, of course. Naturally, molecules that have a molecular skeleton (A-K)ₙ-Y associated to the structural feature or formed by inserting amino acids or chemical groups between A and K are also included in the present invention. (A-K)ₙ-Y is formed by terminating Fmoc at the amino terminal of the peptide molecule with Boc anhydride after Fmoc-Lys (Dde)-OH is condensed to the terminal of the peptide molecule, deprotecting the Dde with 2% Piperidine/DMF, and then condensing and bonding segment A further, so that the skeleton of the peptide compound is reduced by one copy of segment A compared with A-(A-K)ₙ-Y and becomes (A-K)ₙ-Y, which is a variant of A-(A-K)ₙ-Y.

A molecular structure formed by replacing the terminal groups in the n copies of segment A with Fmoc-Lys (Fmoc)-OH is also a variant of A-(A-K)ₙ-Y in the present invention; for example, the same product A-(A-K)ₙ-Y can be obtained by replacing the Fmoc-Lys (Dde)-OH at the terminals of the "Kₙ-Y-solid resin" with Fmoc-Lys (Fmoc)-OH.

In the preparation of the A-(A-K)ₙ-Y molecule that contains multiple copies of segment A, by replacing one or more Fmoc-Lys (Dde)-OH with Fmoc-Lys (Fmoc)-OH, an A₂K-(A-K)-Y molecule that contains three copies of segment A, (A₂K)₂K-(A-K)-Y molecule that contains five copies of segment A, or [(A₂K)₂K]₂K-(A-K)-Y molecule that contains nine copies of segment A can be formed, and those variant structures include the feature of any number of copies of segment A, and also belong to the scope of the present invention, of course.

Hereunder the present invention will be further detailed in embodiments in order to describe the present invention more specifically, but those embodiments should not be understood as constituting any limitation to the present invention. Any modification or derivation made by those skilled in the art to the embodiments in the present invention on the basis of the reveal in this document should be deemed as falling in the scope of the present invention.

### Embodiment 1: Synthesis of copy peptide fragment

The polypeptide compound disclosed in the present invention has the same copied peptide segment A, no matter whether it includes two copies, three copies, or five copies, ..., wherein, A is a short peptide fragment X_{A}X_{B}X_{C}X_{D}-X that has biological activity; wherein, X_{A} and X_{B} are aliphatic amino acid molecules, including any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), and glutamine (Gln, Q), and may be the same or different; X_{C} and X_{D} may be selected from any one of above-mentioned aliphatic amino acid molecules, and may be the same or different; or they may be selected from heterocyclic amino acids, including any of tryptophan (Trp, W), histidine (His, H) and proline (Pro, P), and may be the same or different; K is lysine Fmoc-Lys (Dde)-OH that contain two active amino groups, X or Y is null, or any amino acid, or a peptide fragment composed by any number of amino acids, or a chemical group that can bond up amino acids and peptide fragments; some possible combinations are shown in Table 2, but the present invention is not limited to those combinations.

This embodiment is described exemplarily with synthesis of peptide fragments at free carboxyl terminals, but the peptide and carboxyl terminal may be chemically modified additionally, e.g., by amidation, etc., in actual applications.

The synthesis may be carried out by manual synthesis with solid phase Fmoc/tBu or carried out with an automatic polypeptide synthesizer (Model ABI433A). The specific operations are as follows:
Fmoc-aaₓ-Wang resin (the substitution value is 0.40mmol/g) is used as a starting raw material. Synthesis is carried out from carboxyl terminal to amino terminal, a protecting group, Fmoc, at the amino terminal of the initial amino acid in the resin is removed with 20% Piperidine/DMF (V/V) so that the amino terminal becomes a free amino group (twice, 10min. each time), and Fmoc-aa_{D}-OH/HOBt/DIC in quantity equivalent to three times of equivalent is used to condense with the resin, and the reaction time is 1h. After each step of reaction, the resin is washed with DMF for 6 times, the extent of condensation reaction is detected by Kaiser Test, and the condensation is repeated once more if it is found that the condensation reaction is incomplete. Fmoc-aa_{D}-OH, Fmoc-aa_{C}-OH, Fmoc-aa_{B}-OH, and Fmoc-aa_{A}-OH are condensed sequentially (Fmoc-aa_{D}-OH, Fmoc-aa_{C}-OH, Fmoc-aa_{B}-OH, and Fmoc-aa_{A}-OH represent amino acids X_{D}, X_{C}, X_{B}, and X_{A} in which the amino group is protected by Fmoc) according to the sequence X_{A}X_{B}X_{C}X_{D}-X of segment A, and the reaction cycle is repeated once whenever an amino acid is condensed, till the segment A is synthesized completely.

After the synthesis is completed, the segment A is cracked from the solid resin with 95% TFA/H₂O, and the protecting groups on the side chains are removed (cracked for 3h at 30□). A plenty of cold anhydrous ether is added into the collected liquid that contains the cracked segment A so that the peptide precipitates, then the mixture is centrifuged, the supernatant is removed, and the precipitate is washed with ether for several times and then dried. Thus, a crude product of segment A is obtained.

The crude product of segment A is purified with a high efficiency liquid chromatographic (HPLC) column (Model Daiso C18, 10µm, 100Å, 50×250mm). In the chromatographic operation, the mobile phase A is water solution that contains 0.05% trifluoroacetic acid and 2% acetonitrile, the mobile phase B is 90% acetonitrile/water, the flowing rate is 25mL/min., and the ultraviolet detection wavelength is 220nm. The eluting peak solution is collected, and then a product of segment A peptide at purity>98% is obtained. After freeze drying, a white flocculent segment A peptide product is obtained, and the product is sealed and packed, and stored in a dark and cold environment.

The raw material Fmoc-aa-OH for amino acid synthesis of X_{A}, X_{B}, X_{C} and X_{D} is commercially available. When the segment A for the peptide compound described in the present invention is prepared, X_{D}-WANG resin or X-WANG resin may be directly purchased alternatively, and the peptide segment A copies in the present invention can be obtained with the method described above. Table 1 lists some peptide segment A copies obtained through synthesis, and their molecular weights are measured by mass spectrometry.

**Table 1. List of Groups in Single-Copy Peptide Fragment X_{A}X_{B}X_{C}X_{D}-X**

| #Peptide No. - Number of Copies in the Peptide Fragment Represented by A | X_{A} | X_{B} | X_{C} | X_{D} | X | Molecular Weight |
|---|---|---|---|---|---|---|
| #1-1 | R | K | D | V | Y | 679.76 |
| #2-1 | G | Q | R | P | R | 612.28 |
| #3-1 | T | K | P | P | R | 579.71 |
| #4-1 | K | H | G | K | HG | 662.74 |
| #5-1 | V | K | G | F | Y | 612.72 |
| #6-1 | T | K | P | R | Null | 500.59 |
| #7-1 | N | G | M | T | Y | 584.64 |
| #8-1 | Q | R | P | R | G | 612.68 |
| #9-1 | T | K | L | K | Null | 488.62 |
| #10-1 | T | K | D | L | KEK | 861 |
| #11-1 | R | K | E | V | Y | 693.79 |
| #12-1 | N | G | L | A | P | 470.52 |
| #13-1 | K | E | K | H | G | 579.71 |
| #14-1 | T | K | P | R | KHG | 822.96 |
| #15-1 | H | C | Q | R | PR | 795.92 |

The detected error between the molecular weight of each segment A measured by mass spectrometry in the Table 1 and the theoretical value of molecular weight is within a 0.01% range, which proves that the segment A is right the segment A in the corresponding embodiment.

The Table 1 in this embodiment lists some examples of segment A, but the embodiment is not limited to the segments listed in the Table 1. The synthesis may be carried out in a way as described in the following embodiments, but is not limited to that way.

### Embodiment 2: Synthesis of three-copy A-(A-K)₂-Y peptide molecule and five-copy A-(A-K)₄-Y peptide molecule

In the present invention, the synthesis is described exemplarily with preparation of peptide molecules with free carboxyl terminals. In actual applications, the carboxyl terminals in the synthesized peptide molecules are not limited to that form, and an appropriate solid resin may be selected as required for the synthesis.

The three-copy peptide molecule A-(A-K)₂-Y is a polypeptide compound X_{A}X_{B}X_{C}X_{D}-X-(X_{A}X_{B}X_{C}X_{D}-X-K)₂-Y that contains three copies of X_{A}X_{B}X_{C}X_{D}-X. The structure and the synthetic route are shown in formula 1: 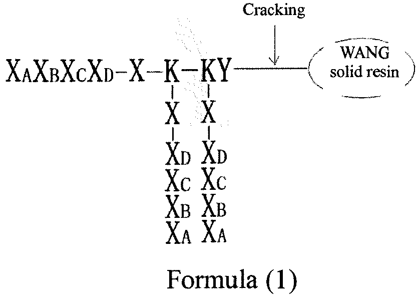 Wherein, X_{A} and X_{B} are aliphatic amino acid molecules, including any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), and glutamine (Gln, Q), and may be the same or different; X_{C} and X_{D} may be selected from any one of above-mentioned aliphatic amino acid molecules, and may be the same or different; or they may be selected from heterocyclic amino acids, including any one of tryptophan (Trp, W), histidine (His, H) and proline (Pro, P), and may be the same or different; K is lysine Fmoc-Lys (Dde)-OH that contain two active amino groups, X or Y is null, or any amino acid, or a peptide fragment composed by any number of amino acids, or a chemical group that can bond up amino acids and peptide fragments.

The method for preparing a three-copy segment A peptide molecule is as follows:
Y-solid resin → enter into "cycle 1" of Fmoc-Lys (Dde)-OH condensation → complete two "cycle 1" →
Fmoc-Lys (Dde)-Lys (Dde)-Y-solid resin → Dde and Fmoc deprotection → H2N-Lys (H₂N)-Lys (H₂N)-Y-solid resin → synthesize segment A - enter into "cycle 2" of Fmoc-aa-OH condensation → A-Lys (A)-Lys (A)-Y-solid resin → complete the synthesis → remove the protecting groups on the side chains and crack the peptide from the resin to obtain a crude product of A-Lys (A)-Lys (A)-Y → purify → fine peptide product → store in a cold environment.

In this embodiment, a solid-phase polypeptide synthesis method is used, and the specific operations are as follows:
In this embodiment, the polypeptide is synthesized manually by condensing and extending amino acids one by one from the carboxyl terminal (C) to the amino terminal (N) of the polypeptide, and then cracking the target polypeptide from the solid resin with a TFA method after the synthesis is finished.

First, Y is fixed to the solid resin, and then two lysine Fmoc-Lys (Dde)-OH molecules, each with two active amino groups, are condensed one by one to obtain K₂-Y resin. Since the lysine at the terminal has an activated amino group, the lysine can react with another lysine (Lys, K) with an active amino group, and then K₂Y-WANG resin with an extended linear chain can be obtained; then, the terminal lysine reacts with segment A (X_{A}X_{B}X_{C}X_{D}-X) at the active amino group, and then X_{A}X_{B}X_{C}X_{D}-X-K₂Y-WANG solid resin with an extended linear chain is obtained; Dde and Fmoc deprotection is carried out for the protected amino groups of lysine on the side chains in the K₂Y-WANG resin with 2% Piperidine/DMF (V/V), and then amino acids X, X_{D}, X_{C}, X_{B}, and X_{A} that constitute segment A (X_{A}X_{B}X_{C}X_{D}-X) are condensed one by one at the free amino groups on the side chains at the same time. Thus, a compound X_{A}X_{B}X_{C}X_{D}-X-(X_{A}X_{B}X_{C}X_{D}-X-K)₂-Y-WANG resin (or A-(A-K)₂-Y-WANG resin), which contains three copies of segment A X_{A}X_{B}X_{C}X_{D}-X, is obtained.

In this step, X_{A}X_{B}X_{C}X_{D}-X segments may be synthesized as described in the embodiment 1 first, and then they may be condensed with the K₂-Y-WANG solid resin; or amino acids X, X_{D}, X_{C}, X_{B} and X_{A} that constitute segment A can be introduced and condensed sequentially at the same time at the free amino groups of the K₂-Y-WANG resin, to obtain A-Lys (A)-Lys (A)-Y-WANG resin. Finally, A-Lys (A)-Lys (A)-Y is cracked from the WANG resin with 95% TFA/H₂O, to obtain a crude product of polypeptide compound A-Lys (A)-Lys (A)-Y or A-(A-K)₂-Y.

Purification of the peptide compound X_{A}X_{B}X_{C}X_{D}-X-(X_{A}X_{B}X_{C}X_{D}-X-K)₂-Y:
The crude product is purified with a chromatographic column (model: Daiso C18, 10µm, 100Å, 50×250mm), wherein, the mobile phase A in the chromatographic operation is water solution that contains 0.05% trifluoroacetic acid and 2% acetonitrile, the mobile phase B is 90% acetonitrile/water, the flowing rate is 25mL/min., and the ultraviolet detection wavelength is 220nm. The eluting peak solution is collected and then freeze-dried. Thus, a white flocculent X_{A}X_{B}X_{C}X_{D}-X-(X_{A}X_{B}X_{C}X_{D}-X-K)₂Y polypeptide compound is obtained. Then, the polypeptide compound is packed in a sealed state and stored in a refrigerator for later use; the purity of the polypeptide compound may be >99%.

The raw material Fmoc-aa-OH for amino acid synthesis of X_{A}, X_{B}, X_{C} and X_{D} is commercially available. When the polypeptide compound in the present invention is prepared, the Y-WANG resin raw material may be directly purchased alternatively, and the polypeptide compound described in the present invention can be obtained with the method described above.
Peptide molecule X_{A}X_{B}X_{C}X_{D}-X-(X_{A}X_{B}X_{C}X_{D}-X-K)₂-Y that contains three copies of segment A and peptide molecule X_{A}X_{B}X_{C}X_{D}-X-(X_{A}X_{B}X_{C}X_{D}-X-K)₄-Y that contains five copies of segment A are synthesized with the exemplary method in this embodiment. The selection of the specific groups is shown in Table 2 and Table 3 respectively.

**Table 2. List of Groups in the Peptide Molecule X_{A}X_{B}X_{C}X_{D}-X-(X_{A}X_{B}X_{C}X_{D}-X-K)₂-Y Containing Three Copies of Segment A**

| #Peptide No. - Number of Copies in the Peptide Fragment Represented by A | X_{A} | X_{B} | X_{C} | X_{D} | X | Y | Molecular Weight |
|---|---|---|---|---|---|---|---|
| #1-3 | R | K | D | V | Y | G | 2316.66 |
| #2-3 | G | Q | R | P | R | Null | 2058.36 |
| #3-3 | T | K | P | P | R | G | 2070.49 |
| #4-3 | K | H | G | K | HG | G | 2265.59 |
| #5-3 | V | K | G | F | Y | Null | 2058.46 |
| #6-3 | T | K | P | R | Null | G | 1779.14 |
| #7-3 | N | G | M | T | Y | Null | 1974.25 |
| #8-3 | Q | R | P | R | G | A | 2129.44 |
| #9-3 | T | K | L | K | Null | G | 1743.23 |
| #10-3 | T | K | D | L | KEK | G | 2860.35 |
| #11-3 | R | K | E | V | Y | Null | 2301.69 |
| #12-3 | N | G | L | A | P | G | 1688.93 |
| #13-3 | K | E | K | H | G | Null | 2013.31 |
| #14-3 | T | K | P | R | KHG | G | 2746.23 |
| #15-3 | H | C | Q | R | PR | Null | 2608.06 |

The detected error between the molecular weight of each three-copy segment A peptide molecule measured by mass spectrometry in the Table 2 and the theoretical value of molecular weight is within a 0.01% range, which proves that the three-copy segment A peptide molecule is right the peptide molecule in the corresponding embodiment.

This embodiment only describes the segment A and the form of the molecular structure formed here exemplarily, and is not limited to the listed segments. The above content doesn't constitute any limitation to the present invention, and the synthesis may be carried out actually in the way described in the following embodiments, but is not limited to that way.

### Embodiment 3: Synthesis of five-copy peptide molecule X_{A}X_{B}X_{c}X_{D}-X-(X_{A}X_{B}X_{C}X_{D}-X-K)₄-Y

The five-copy peptide molecule A-(A-K)₄-Y provided in the present invention is a polypeptide compound X_{A}X_{B}X_{C}X_{D}-X-(X_{A}X_{B}X_{C}X_{D}-X-K)₄-Y that contains five copies of X_{A}X_{B}X_{C}X_{D}-X. The structure and the synthetic route are shown in formula 2: wherein, X_{A} and X_{B} are aliphatic amino acid molecules, including any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), and glutamine (Gln, Q), and may be the same or different; X_{C} and X_{D} may be selected from any one of above-mentioned aliphatic amino acid molecules, and may be the same or different; or they may be selected from heterocyclic amino acids, including any of tryptophan (Trp, W), histidine (His, H) and proline (Pro, P), and may be the same or different; K is lysine Fmoc-Lys (Dde)-OH that contain two active amino groups, X or Y is null, or any amino acid, or a peptide fragment composed by any number of amino acids, or a chemical group that can bond up amino acids and peptide fragments:

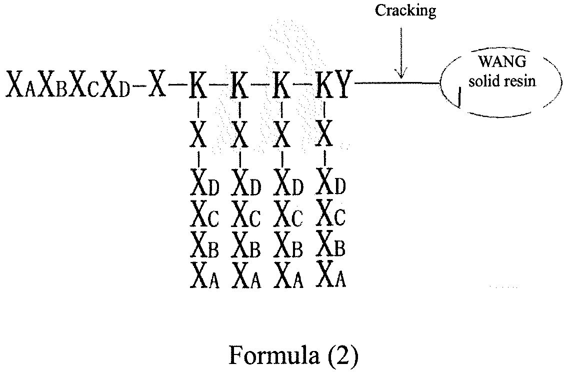

The method for preparing a five-copy segment A peptide molecule is as follows:
Y-solid resin → enter into "cycle 1" of Fmoc-Lys (Dde)-Lys (Dde)-OH condensation → complete four "cycle 1" → Fmoc-Lys (Dde)-Lys (Dde)-Lys (Dde)-Lys (Dde)-Y-solid resin → Dde and Fmoc deprotection for amino groups on side chains → H₂N-Lys (H₂N)-Lys (H₂N)-Lys (H₂N)-Lys (H₂N)-Y-solid resin → synthesize segment A - enter into "cycle 2" of Fmoc-aa-OH condensation → A-Lys (A)-Lys (A)-Lys (A)-Lys (A)-Y-solid resin → complete the synthesis → remove the protecting groups on the side chains and crack the peptide from the resin to obtain a crude product of A-Lys (A)-Lys (A)-Lys (A)-Lys (A)-Y → purify → fine peptide product → store in a cold environment.

In this embodiment, a solid-phase polypeptide synthesis method is used, and the specific operations are as follows:
Synthesis of polypeptide compound X_{A}X_{B}X_{C}X_{D}-X-(X_{A}X_{B}X_{C}X_{D}-X-K)₄-Y:
In this embodiment, the polypeptide is synthesized manually by condensing and extending amino acids one by one from the carboxyl terminal (C) to the amino terminal (N) of the polypeptide, and then cracking the target polypeptide from the solid resin with a TFA method after the synthesis is finished.

First, Y is fixed to the solid resin, and then four lysine Fmoc-Lys (Dde)-OH molecules, each with two active amino groups, are condensed one by one to obtain K₄-Y resin. Since the lysine at the terminal has an activated amino group, the lysine can react with another lysine (Lys, K) with an active amino group, and the react with third and fourth lysine (Lys, K) with an active amino group, and then K₄Y-WANG resin with an extended linear chain can be obtained; then, the terminal lysine reacts with segment A (X_{A}X_{B}X_{C}X_{D}-X) at the active amino group, and then X_{A}X_{B}X_{C}X_{D}-X-K₄-Y-WANG solid resin with an extended linear chain is obtained; Dde and Fmoc deprotection is carried out for the amino groups of lysine side chains in the K₄Y-WANG resin with 2% Piperidine/DMF (V/V); and then amino acids X, X_{D}, X_{C}, X_{B}, and X_{A} that constitute the segment A are introduced and condensed at the free amino groups in the K₄Y-WANG resin at the same time, i.e., after lysine (Lys, K) deprotection, segments A (X_{A}X_{B}X_{C}X_{D}-X) are condensed to the free amino groups, and then a compound X_{A}X_{B}X_{C}X_{D}-X-(X_{A}X_{B}X_{C}X_{D}-X-K)₄-Y-WANG resin (or A-(A-K)₄-Y-WANG resin) that contains five copies of segment A X_{A}X_{B}X_{C}X_{D}-X is obtained.

Finally, the peptide compound A-(A-K)₄-Y is cracked from the WANG resin with 95% TFA/H₂O, to obtain a crude product of X_{A}X_{B}X_{C}X_{D}-X- (X_{A}X_{B}X_{C}X_{D}-X-K)₄Y (or A-(A-K)₄-Y).

Purification of polypeptide compound A-(A-K)₄-Y:
The crude product is purified with a chromatographic column (model: Daiso C18, 10µm, 100Å, 50×250mm), wherein the mobile phase A in the chromatographic operation is water solution that contains 0.05% trifluoroacetic acid and 2% acetonitrile, the mobile phase B is 90% acetonitrile/water, the flowing rate is 25mL/min., and the ultraviolet detection wavelength is 220nm. The eluting peak solution is collected and then freeze-dried. Thus, a white flocculent A-(A-K)₄-Y polypeptide compound is obtained. Then, the polypeptide compound is packed in a sealed state and stored in a refrigerator for later use; the purity of the polypeptide compound may be >99%.

The raw materials X_{A}, X_{B}, X_{C} and X_{D} that are used to synthesize the segment A are commercially available. When the polypeptide compound in the present invention is prepared, Y-WANG resin may be directly purchased as a starting raw material for the synthesis, and amino acids may be introduced and condensed to the amino terminal of Y with the above-mentioned method, so as to obtain the polypeptide compound in the present invention.

A series of five-copy peptide molecules X_{A}X_{B}X_{C}X_{D}-X-(X_{A}X_{B}X_{C}X_{D}-X-K)₄-Y are obtained with the method described in this embodiment. Please see Table 3 for the selection of the groups.

**Table 3. List of Groups in the Peptide Molecule X_{A}X_{B}X_{C}X_{D}-X-(X_{A}X_{B}X_{C}X_{D}-X-K)₄-Y Containing Five Copies of Segment A**

| #Peptide segment No. - Number of Copies in the Peptide Fragment Represented by A | X_{A} | X_{B} | X_{C} | X_{D} | X | Y | Molecular Weight |
|---|---|---|---|---|---|---|---|
| #1-5 | R | K | D | V | Y | G | 3896.5 |
| #2-5 | G | Q | R | P | R | Null | 3504.04 |
| #3-5 | T | K | P | P | R | G | 3486.22 |
| #4-5 | K | H | G | K | HG | G | 3811.39 |
| #5-5 | V | K | G | F | Y | Null | 3504.21 |
| #6-5 | T | K | P | R | Null | G | 3000.64 |
| #7-5 | N | G | M | T | Y | Null | 3363.85 |
| #8-5 | Q | R | P | R | G | A | 3575.12 |
| #9-5 | T | K | L | K | Null | G | 2940.79 |
| #10-5 | T | K | D | L | KEK | G | 4802.66 |
| #11-5 | R | K | E | V | Y | Null | 3909.59 |
| #12-5 | N | G | L | A | P | G | 2850.28 |
| #13-5 | K | E | K | H | G | Null | 3429.17 |
| #14-5 | T | K | P | R | KHG | G | 4612.46 |
| #15-5 | H | C | Q | R | PR | Null | 4420.21 |

The detected error between the molecular weight of each five-copy segment A peptide molecule measured by mass spectrometry in the Table 3 and the theoretical value of molecular weight is within 0.01% range, which proves that the five-copy segment A peptide molecule is right the peptide molecule in the corresponding embodiment.

This embodiment only describes the segment A and the form of the molecular structure formed here exemplarily, and is not limited to the listed segments. The above content doesn't constitute any limitation to the present invention, and the synthesis may be carried out actually in the way described in the following embodiments, but is not limited to that way.

Peptide molecules that contain any number of copies of biologically active peptide segment can be prepared with the method described in the present invention. In the embodiments, typical peptide molecule that contains three copies of biologically active peptide segment (segment A) and peptide molecule that contains five copies of biologically active peptide segment are prepared and tested particularly, to prove the feasibility and effectiveness of the preparation of peptide molecule A-(A-K)ₙ-Y described in the present invention.

The present invention relates to preparation of compounds that contain multiple copies of a biologically active peptide segment. In the molecular formula A-(A-K)ₙ-Y, A represents the biologically active peptide segment. For illustration purpose, some biologically active segments employed in the embodiments are listed in the tables. However, in actual applications, A represents all peptide fragments that have biological activity, and the biological functions involved here are not only limited to those of the active peptide segments that are described exemplarily in the embodiments and have a limited range of functions.

All peptide compound molecules that are derived by adding or reducing amino acids, replacing amino acids, modifying amino acid terminals, or modifying amino acid side chains for known active peptide segment A on the basis of the biologically functional segment disclosed in the embodiments, and all molecules prepared according to the A-(A-K)ₙ-Y peptide molecular structure, should be deemed as falling in the scope of the present invention. Hereunder the biological activity and efficacies of the polypeptide compound provided in the present invention will be described in specific experimental examples.

### Experimental example 1: Experiment of immune effect of the polypeptide compound provided in the present invention among birds and poultries (chicks)

Red blood cells in human or animal body may be agglutinated by viruses that contain hemagglutinin (HA), and that phenomenon is referred to as a hemagglutination phenomenon. Such a hemagglutination phenomenon can be inhibited by a specific immune serum or a specific antibody, and the inhibition test is referred to as "hemagglutination inhibition test" (HI).

Newcastle disease virus (NDV) can cause an agglutination phenomenon of chicken red blood cells. After chicken is immunized with a Newcastle disease virus vaccine, the level of antibody can be judged by a hemagglutination inhibition test (HI) in which chicken serum is tested. The maximum multiple of dilution of the anti-serum for the test is the titer of the antibody. The higher the titer of the tested antibody is, the better the immune effect is.

The HI method has the following advantages:
1. High sensitivity: The HI method can detect antibody in a trace quantity; the result is relatively accurate; and the reaction is one of sensitive serologic detection reactions;
2. High specificity: A virus that causes agglutination of red blood cells can only be inhibited by a specific antibody;
3. High detection speed: Only about 2h is required in a HI test to judge the result;
4. The HI test doesn't have any high requirement for the environment; the operation is simple and quick; and a large quantity of samples can be detected in one test.

In this experimental example, different three-copy segments in the Table 2 are used to investigate the influence of multi-copy biologically active peptide molecules used in combination with the vaccine on the generation of antibody in living body.

The experimental method is as follows: specific pathogen free chicks aged 10 days (abbreviated as SPF chicks) were chosen. The SPF chicks were divided into 12 groups, with 10 chicks in each group. Subcutaneous vaccination was carried out in the axillary region of a wing of each SPF chick in the following groups. The SPF chicks in each group were bred in isolators. About 1ml of vein blood was taken under the wings on the tenth day after inoculation. The serum was separated from the blood and was tested by an HI test. The test results are shown in Table 4 (only the test results of a part of the polypeptide compounds are listed). See the "Experiment Course of Animal Immunology" authored by Xin Guo and published by the Press of China Agricultural University in 2007 for the details of operation.
Blank group: 0.3ml normal saline was injected;
Control group: 0.3ml of live vaccine of Newcastle disease (abbreviated as "the vaccine", CS2 strain, from Chengdu Tecbond Biological Products Co., Ltd.) was inoculated;
Experimental group: 0.3ml of vaccine mixed with 0.2µg of polypeptide compound provided in the present invention was inoculated.

The groups of SPF chicks were bred normally during the experiment under the same feeding and management conditions. The dietetic activities of the SPF chicks in the groups were normal; no adverse reaction was seen; and no SPF chick died. That indicates the polypeptide compound provided in the present invention is safe to use.

The results in Table 4 indicate that the average antibody titer of HI is improved after the polypeptide compound described in the present invention is added to the chicken vaccine, when compared with the values in the blank group and the control group (vaccine solely). That proves the polypeptide compound provided in the present invention attains a good immune improvement effect when it is used in combination with the vaccine.

**Table 4. Result of Immunity Experiment of SPF Chicks**

| Group | Inoculated Substance | Average Antibody Titer | Group | Inoculated Substance | Average Antibody Titer |
|---|---|---|---|---|---|
| Blank group | Normal saline | Negative | | | |
| Control group | Vaccine | 8.2log2 | Experimental group 8 | Vaccine+# 8-3 | 9.0log2 |
| Experimental group 1 | Vaccine+#1-3 | 8.9log2 | Experimental group 9 | Vaccine+# 9-3 | 9.2log2 |
| Experimental group 2 | Vaccine+#2-3 | 9.0log2 | Experimental group 10 | Vaccine+# 10-3 | 9.1log2 |
| Experimental group 3 | Vaccine+#3-3 | 9.2log2 | Experimental group 11 | Vaccine+# 11-3 | 9.1log2 |
| Experimental group 4 | Vaccine+#4-3 | 9.2log2 | Experimental group 12 | Vaccine+# 12-3 | 8.8log2 |
| Experimental group 5 | Vaccine+#5-3 | 9.1log2 | Experimental group 13 | Vaccine+# 13-3 | 9.2log2 |
| Experimental group 6 | Vaccine+#6-3 | 9.2log2 | Experimental group 14 | Vaccine+# 14-3 | 9. 1log2 |
| Experimental group 7 | Vaccine+#7-3 | 8.8log2 | Experimental group 15 | Vaccine+# 15-3 | 9.1log2 |

| | | | | | |
|---|---|---|---|---|---|
| Note: "Negative" refers to that the HI antibody titer is zero. | | | | | |

### Experimental example 2: Experiment of comparison between influences of peptide molecules that contain different number of copies of biologically active segments on immune effect

A biologically active peptide molecule, a three-copy active peptide molecule, and a five-copy active peptide molecule were selected randomly in the experimental example 1, and the influences of the active peptide molecules that contained different number of copies of active peptide segments on the immune effect were tested. The test results are shown in Table 5.

Group division:
Blank group: 0.3ml of normal saline was injected;
Control group: 0.3ml of live vaccine of Newcastle disease (abbreviated as "the vaccine", CS2 strain) was inoculated to each chick according to the specification provided by the vaccine manufacturer;
Experimental group: 0.3ml of vaccine mixed with 0.2µg of peptide was inoculated to each chick according to the specification provided by the vaccine manufacturer;

**Table 5. Result of Immunity Experiment of SPF Chicks**

| Group | Inoculated Substance | Average Antibody Titer | Group | Inoculated Substance | Average Antibody Titer |
|---|---|---|---|---|---|
| Blank group | Normal saline | Negative | Control group | Vaccine | 8.2log2 |
| Experimental group #4-1 | Vaccine+#4-1 | 8.6log2 | Vaccine+#11-1 | Vaccine+#9-1 | 8.6log2 |
| Experimental group #4-3 | Vaccine+#4-3 | 9.2log2 | Vaccine+#11-3 | Vaccine+#9-3 | 9.2log2 |
| Experimental group #4-5 | Vaccine+#4-5 | 9.6log2 | Vaccine+#11-5 | Vaccine+#9-5 | 9.6log2 |
| Experimental group #6-1 | Vaccine+#6-1 | 8.7log2 | Vaccine+#13-1 | Vaccine+#13-1 | 8.6log2 |
| Experimental group #6-3 | Vaccine+#6-3 | 9.3log2 | Vaccine+#13-3 | Vaccine+#13-3 | 9.2log2 |
| Experimental group #6-5 | Vaccine+#6-5 | 9.6log2 | Vaccine+#13-5 | Vaccine+#13-5 | 9.5log2 |

| | | | | | |
|---|---|---|---|---|---|
| Note: "Negative" refers to that the HI antibody titer is zero. | | | | | |

The dietetic activities of the SPF chicks in the groups were normal during the experiment; no adverse reaction was seen; and no SPF chick died. That indicates the polypeptide compound provided in the present invention is safe to use. The results in Table 5 indicate that the immune improvement effect is further increased as the number of copies of peptide fragment is increased. That means there is a positive correlation between the biological effect and the number of copies of peptide fragment.

### Experimental example 3: Experiment on solid tumor inhibition effect of peptide compounds that contain different number of copies of peptide segment

Objective of experiment: To test the inhibition effect of synthetic peptide samples that contain different number of copies of peptide segments on solid tumors in mice

Tested samples: Synthetic peptide samples #6-1, #6-3, and #6-5, all of which were freeze-dried samples in 5mg/vial specification, at purity>98.5%, stored at -20°C in a refrigerator; the samples were diluted on the spot with water for injection as 2mg/ml, and were injected by hypodermic injection to mice at the nape; the remaining sample was discarded after each injection.

Positive control sample: 5-fluorouracil (abbreviated as 5-FU), from Tianjin Kingyork Amino Acids Co., Ltd., in 250mg/10mL/vial specification, with lot number: 1600108; the medicine was injected to the mice at the abdomen, and the remaining medicine was discarded after each injection.

Tested animals: Mus Musculus Balb/c female mice, 6-8 weeks aged, with 23±2g body weight. The tested animals were from Beijing Huabukang Biological Technology Co., Ltd.

The mice were bred in a SPF-level constant-temperature, constant-humidity and laminar flow poultry house at 22±3°C temperature and 40-80% relative humidity, with 5 mice kept in each cage; the mice took clean-grade mouse feed blocks and drank filtered and sterilized water freely, and were managed according to the mouse breeding rules for the poultry house.

Test method: Hepatoma 22 (H22) tumor cells cultured in vitro in the logarithmic growth phase were used for the inoculation, the suspended tumor cells were diluted with PBS buffer solution to 1×10⁶ piece/0.1ml concentration, and then were inoculated by subcutaneous injection to the mice at the right shoulder blade. The condition of tumor growth in the mice was observed closely, and the medicines were administered to the mice in groups when the tumors under the skins of the mice grew to about 50-80mm³.

Termination of experiment: The tumor volumes in the mice in the groups were measured by measuring the major diameter and minor diameter of the tumor with verniers in every two days. All of the experimental animals were killed by means of neck dislocation when the average tumor volume in the control group reached 2,000m³, the tumor tissues were peeled off and weighed, and the weight of each tumor and average tumor weight in each group are recorded. The tumor inhibition rate was calculated according to the average tumor weight in each group, i.e., tumor inhibition rate (%) = (1 - average tumor weight in the sample group / average tumor weight in the blank group) × 100%. One-way ANOVA verification was carried out SPSS vl3.0 statistical software. The results are shown in Table 6.

**Table 6. Result of Test on Inhibition of Hepatoma 22 (H22) Solid Tumors in Mice**

| Group | Mice | Dose | Administration method | Course of treatment | Tumor weig ht (g) | Tumor inhibition rate (%) | P^{b} |
|---|---|---|---|---|---|---|---|
| Blank group (normal saline) | 8 mice | 0.25ml | Subcutaneous injection | 1 time/day, 14 days | 2.346±0.312 | -- | -- |
| Positive medicine group (5-FU) | 8 mice | 20mg/kg | Intraperitoneal injection | 1 time/day, 5 days | 1.135±0.248 | 51.62% | <0.05 |
| Sample group #6-1 | 8 mice | 20mg/kg | Subcutaneous injection | 1 time/day, 14 days | 1.986±0.244 | 15.35% | >0.05 |
| Sample group #6-3 | 8 mice | 20mg/kg | Subcutaneous injection | 1 time/day, 14 days | 1.729±0.252 | 26.30% | >0.05 |
| Sample group #6-5 | 8 mice | 20mg/kg | Subcutaneous injection | 1 time/day, 14 days | 1.587±0.312 | 32.35% | <0.05 |

### Analysis of the results:

Through comparison between the average tumor weight in the sample group and the average tumor weight in the blank group, it is found that the tumor inhibition effect is improved as the number of copies of active peptide is increased.

### Experimental example 4: Experiment on the tumor inhibition effect of biologically active peptide compounds that contains 5 copies of peptide segment

Objective of experiment: Three biologically active peptide compounds that contain 5 copies of peptide segments were selected randomly, and their inhibition effects on Hepatoma 22 (H22) solid tumors were tested.

Tested samples: Synthetic peptide samples #4-5, #9-5, and #14-5, all of which were freeze-dried samples in 5mg/vial specification, at purity>98.5%, stored at -20°C in a refrigerator; the samples were diluted on the spot with water for injection as 2mg/ml, and were injected by hypodermic injection to mice at the nape; the remaining sample was discarded after each injection.

Positive control sample: 5-fluorouracil (abbreviated as 5-FU), from Tianjin Kingyork Amino Acids Co., Ltd, in 250mg/10mL/vial specification, with lot number: 1600108; the medicine was injected to the mice at the abdomen, and the remaining medicine was discarded after each injection.

Tested animals: Mus Musculus Balb/c female mice, 6-8 weeks aged, with 23±1g body weight. The tested animals were from Beijing HFK Bioscience Co., Ltd..

The mice were bred in a SPF-level constant-temperature, constant-humidity and laminar flow poultry house at 22±3°C temperature and 40-80% relative humidity, with 5 mice kept in each cage; the mice took clean-grade mouse feed blocks and drank filtered and sterilized water freely, and were managed according to the mouse breeding rules for the poultry house.

Test method: Hepatoma 22 (H22) tumor cells cultured in vitro in the logarithmic growth phase are used for the inoculation, the suspended tumor cells were diluted with PBS buffer solution to 1x10⁶ piece/0.1ml concentration, and then were inoculated by subcutaneous injection to the mice at the right shoulder blade. The condition of tumor growth in the mice was observed closely, and the medicines were administered to the mice in groups when the tumors under the skins of the mice grew to about 50-80mm³.

Termination of experiment: The tumor volumes in the mice in the groups were measured by measuring the major diameter and minor diameter of the tumor with verniers in every two days. All of the experimental animals were killed by means of neck dislocation when the average tumor volume in the control group reaches 2,000m³, the tumor tissues were peeled off and weighed, and the weight of each tumor and average tumor weight in each group were recorded. The tumor inhibition rate was calculated according to the average tumor weight in each group, i.e., tumor inhibition rate (%) = (1 - average tumor weight in the sample group / average tumor weight in the blank group) x 100%. One-way ANOVA verification was carried out SPSS v13.0 statistical software. The results are shown in Table 7.

**Table 7. Result of Test on Inhibition of Hepatoma 22 (H22) Solid Tumors in Mice**

| Group | Mice | Dose | Administrati on method | Course of treatment | Average tumor weight (g) | Tumor inhibition rate (%) | P^{b} |
|---|---|---|---|---|---|---|---|
| Blank group (normal saline) | 8 mice | 0.25 ml | Subcutaneous injection | 1 time/day, 14 days | 2.346±0.312 | -- | -- |
| Positive medicine group (5-FU) | 8 mice | 20mg/kg | Intraperitoneal injection | 1 time/day, 5 days | 1.135+0.248 | 51.62% | <0.05 |
| Sample group #4-5 | 8 mice | 20mg/kg | Subcutaneous injection | 1 time/day, 14 days | 1.558±0.351 | 33.58% | <0.05 |
| Sample group #9-5 | 8 mice | 20mg /kg | Subcutaneous injection | 1 time/day, 14 days | 1.547±0.323 | 34.05% | <0.05 |
| Sample group #14-5 | 8 mice | 20mg /kg | Subcutaneous injection | 1 time/day, 14 days | 1.553±0.412 | 33.80% | <0.05 |

### Analysis of the results:

Through comparison between the average tumor weight in the sample group and the average tumor weight in the blank group and statistical analysis, the result indicates P<0.05, which proves that there is a significant difference in Hepatoma 22 (H22) tumor inhibition effect among the examples #4-5, #9-5 and #14-5.

### Industrial Applicability

The polypeptide compound provided in the present invention is hopeful to become an effective ingredient in a variety of medicines, and is applicable to medicines for preventing and treating many diseases. Especially, the polypeptide compound can be used to prepare medicines for enhancing immune ability, and is suitable for industrial application.

## Claims

1. A polypeptide compound represented by a structural formula A-(A-K)n-Y, n being a natural number;
wherein, A is a short peptide fragment with biological activity; K is lysine Fmoc-Lys (Dde)-OH that contains two active amino groups, n is the number of K, Y is null or any one or more amino acids or chemical groups; when n=1, the structural formula of the polypeptide compound is A-(A-K)-Y; when n=2, the structural formula of the polypeptide compound is A-(A-K)₂-Y; if n=3, the structural formula of the polypeptide compound is A-(A-K)₃-Y; ...;
A preferably is a short peptide fragment X_{A}X_{B}X_{C}X_{D}-X; if A is X_{A}X_{B}X_{C}X_{D}-X, the structural formula of the polypeptide compound is X_{A}X_{B}X_{C}X_{D}-X-(X_{A}X_{B}X_{C}X_{D}-X-K)ₙ-Y; wherein, X_{A} and X_{B} are one or two of aliphatic amino acids, X_{C} and X_{D} are selected from one or two of aliphatic amino acids and aromatic heterocyclic amino acids, and X is null or any one or more amino acids or chemical groups.

2. The polypeptide compound according to claim 1, **characterized in that** when n=2 and A is X_{A}X_{B}X_{C}X_{D}-X, the structural formula of the polypeptide compound is X_{A}X_{B}X_{c}X_{D}-X-(X_{A}X_{B}X_{c}X_{D}-X-K)₂-Y, as shown in formula 3:

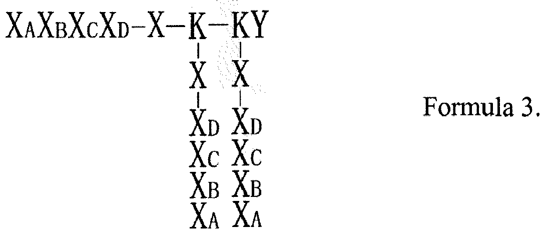

3. The polypeptide compound according to claim 1, **characterized in that** when n=4 and A is X_{A}X_{B}X_{C}X_{D}-X, the structural formula of the polypeptide compound is X_{A}X_{B}X_{C}X_{D}-X-(X_{A}X_{B}X_{C}X_{D}-X-K)₄-Y, as shown in formula 4:

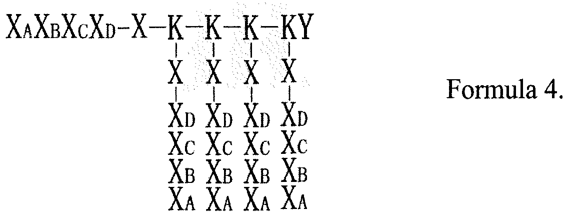

4. The polypeptide compound according to claim 1, 2 or 3, **characterized in that** X or Y is null, or any amino acid, or a peptide fragment composed of any number of amino acids, or a chemical group that connects amino acids or peptide fragments, and X and Y are the same or different from each other; for example, X is null, and Y is glycine (Gly, G).

5. The polypeptide compound according to any one of claims 1-4, **characterized in that** X_{A} and X_{B} are aliphatic amino acid molecules, including any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), and glutamine (Gln, Q), and may be the same or different.

6. The polypeptide compound according to any one of claims 1-5, **characterized in that** X_{C} and X_{D} are selected from any one of phenylalanine (Phe, F), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparaginate (Asn, N), glutamate (Glu, E), glutamine (Gln, Q), tryptophan (Trp, W), histidine (His, H) and proline (Pro, P), and may be the same or different.

7. The polypeptide compound according to any of claims 1-6, further comprising a salt compound formed by the polypeptide compound with an organic acid or inorganic acid.

8. The polypeptide compound according to any one of claims 1-6, further comprising an ether, ester, glucoside, or glycoside compound, etc., which is formed by the hydroxyl included in the polypeptide compound, but is not limited to compounds formed in such a way.

9. The polypeptide compound according to any one of claims 1-6, further comprising a thioether or thioglycoside compound, which is formed by the sulfhydryl included in the polypeptide compound, or a compound containing disulfide bonds, which is formed by the sulfhydryl included in the polypeptide compound with cysteine or peptide containing cysteine, but is not limited to compounds formed in such a way.

10. The polypeptide compound according to any one of claims 1-6, further comprising an acylate or alkylate compound, which is formed by the amido included in the polypeptide compound, or a glucoside compound, etc., which is formed by the amido included in the polypeptide compound with saccharides, but is not limited to compounds formed in such a way.

11. The polypeptide compound according to any one of claims 1-6, further comprising an ester or amide compound, etc., which is formed by the carboxyl included in the polypeptide compound, but is not limited to compounds formed in such a way.

12. The polypeptide compound according to any one of claims 1-6, further comprising a glucoside, acylate, or alkylate compound, etc., which is formed by the imino included in the polypeptide compound, but is not limited to compounds formed in such a way.

13. The polypeptide compound according to any one of claims 1-6, further comprising an ester, ether, glucoside, or glycoside compound, which is formed by the phenolic hydroxyl included in the polypeptide compound, or a salt compound, which is formed by the phenolic hydroxyl included in the polypeptide compound with organic alkalis or inorganic alkalis, but is not limited to compounds formed in such a way.

14. The polypeptide compound according to any one of claims 1-6, further comprising a coordinate, clathrate, or chelate compound formed by the polypeptide compound with metal ions.

15. The polypeptide compound according to any one of claims 1-6, further comprising a hydrate or solvent formed by the polypeptide compound.

16. A pharmaceutical composition, containing the polypeptide compound according to any one of claims 1-15, a geometrical isomer thereof, a pharmaceutically acceptable salt or solvated compound thereof, and a pharmaceutical composition in a form of pharmaceutical carrier or excipient.

17. A method for preparing the polypeptide compound according to any one of claims 1-15, **characterized in that** a synthetic route of A-(A-K)ₙ-Y is shown in formula 5, 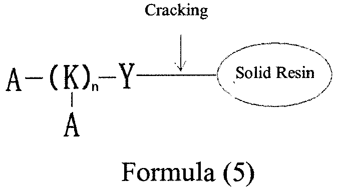
A preferably is a short peptide fragment X_{A}X_{B}X_{C}X_{D}-X; when A is X_{A}X_{B}X_{C}X_{D}-X, the structural formula of the polypeptide compound is X_{A}X_{B}X_{C}X_{D}-X-(X_{A}X_{B}X_{C}X_{D}-X-K)ₙ-Y; wherein, X_{A} and X_{B} are one or two of aliphatic amino acids, X_{C} and X_{D} are selected from one or two of aliphatic amino acids and aromatic heterocyclic amino acids, and X is null or any one or more amino acids or chemical groups;
first, Y is fixed to a solid resin to obtain Y-solid resin; the Y-solid resin is treated by Fmoc deprotection and n pieces Fmoc-Lys (Dde)-OH are condensed one by one, to accomplish preparation of a Kₙ-Y-solid resin peptide skeleton; then, the Kₙ-Y-solid resin peptide skeleton is treated by deprotection of the side chain amino groups, Dde and Fmoc, and segment A extension and introduction is carried out synchronously on the free amino groups of Kₙ-Y-solid resin, to obtain A-(A-K)ₙY-solid resin; after the synthesis is completed, A-(A-K)ₙY is cracked from the solid resin to obtain a crude peptide product, and the crude peptide product is purified by high efficiency liquid chromatography to obtain the polypeptide compound A-(A-K)ₙY.

18. The method according to claim 17, **characterized in that** an appropriate resin for preparation is selected according to the characteristics of the carboxyl terminal of the peptide product; "WANG resin" is selected if the carboxyl terminal of the peptide product is a free carboxyl group; "Rink resin" is selected if the carboxyl terminal of the synthetic peptide is an amido group; "CTC resin" is selected if the carboxyl terminal of the peptide product is Cys, Pro, or His.

19. The method according to claim 17 or 18, **characterized in that** the polypeptide compound is prepared with a solid-phase polypeptide synthesis method, which comprises the following steps:
Step 1: preparing Y-solid resin: if Y is a single amino acid, Y-solid resin is purchased directly;
if Y is a short peptide that contains a plurality of amino acids, a solid phase Fmoc/tBu method is used, i.e., Fmoc-aa₁-Wang resin is used as a starting raw material, synthesis is carried out from the carboxyl terminal (C) to the amino terminal (N), the protecting group, Fmoc, at the amino terminal is removed so that the amino terminal becomes a free amino group, and then the amino terminal is condensed with resin, till Y-solid resin is obtained;
Step 2: preparing Kₙ-Y-solid resin peptide skeleton: the Y-solid resin is treated by Fmoc deprotection to expose the free amino group, and condensation is carried out with Fmoc-Lys (Dde)-OH (the condensation method is the same as that in the step 1); the condensation is repeated till n pieces of Fmoc-Lys (Dde)-OH are condensed (denoted as "cycle 1"), and thereby a Kₙ-Y-solid resin peptide skeleton is prepared;
Step 3: carrying out amino deprotection for the Kₙ-Y-solid resin: the Dde of side chain Lys and the protecting group, Fmoc, in the Kₙ-Y-solid resin peptide skeleton are removed, to obtain a Kₙ-Y-solid resin in which the free amino groups on the side chains are liberated;
Step 4: synthesizing segments A synchronously on the free amino groups of Kₙ-Y-solid resin: the amino acids are condensed one by one with the method describe in the step 1, till segments A are completed and A-(A-K)ₙY-solid resin is obtained (denoted as "cycle 2");
Step 5: cracking the synthesized A-(A-K)ₙY from the solid resin, and removing protecting groups on the side chains, to obtain a crude product A-(A-K)ₙY;
Step 6: purifying to obtain a fine product: the crude peptide is purified by high efficiency liquid chromatography (HPLC), to obtain the polypeptide compound A-(A-K)ₙY at purity>98%.

20. The method according to claim 19, **characterized in that** whenever an amino acid is introduced and condensed, and each time after the amino acid is condensed between the "cycle 1" and the "cycle 2", a "Kaiser Test" is carried out to detect the content of free amino groups, and condensation is repeated once more if the condensation rate of the reaction is not high enough.

21. An application of the polypeptide compound according to any one of claims 1-15 or a polypeptide compound prepared with the method according to any one of claims 17-20 in preparation of medicines for inhibiting tumor growth in human or animal bodies.

22. The application according to claim 21, **characterized in that** the tumor is a a solid tumor (or a residual tumor after surgical operation) or a non-solid tumor in a human body.

23. The application according to claim 22, **characterized in that** the solid tumor (or residual tumor after surgical operation) in human body includes, but is not limited to sarcoma, liver cancer, colon cancer, lung cancer, stomach cancer, mammary cancer, and cervical cancer, and the non-solid tumor is a hematologic tumor (including leukaemia and lymphomata), for example.

24. An application of the polypeptide compound according to any one of claims 1-15 or a polypeptide compound prepared with the method according to any one of claims 17-20 in preparation of immune medicines or medicines for improving immune function for humans or animals.
